# EUROPEAN PATENT APPLICATION

(11) **EP 4 269 391 A1**
(43) Date of publication of application: **01.11.2023**
(21) Application number: 21909296.2
(22) Date of filing: 17.12.2021
(51) Int. Cl.: C07D 211/74, C07D 211/56

(54) **SYNTHESIS AND USE OF 1-BENZYL-4-METHYL-5-ALKOXY-1,2,3,6-TETRAHYDROPYRIDINE DERIVATIVE**

(30) Priority: 22.12.2020 CN 202011526908
(71) Applicant: Zhejiang Ausun Pharmaceutical Co., Ltd., Taizhou, Zhejiang 317016 (CN)
(72) Inventor: LI, Yunfeng, Linhai, Zhejiang 317016 (CN); ZHENG, Zhiguo, Linhai, Zhejiang 317016 (CN)
(74) Representative: Bianchetti & Minoja with Trevisan & Cuonzo IPS SRL
(86) International application number: PCT/CN2021/139218
(87) International publication number: WO 2022/135300

(57) **Abstract**

The invention relates to the field of synthesis of drug intermediates, in particular to the field of synthesis of key intermediates for preparing anti-rheumatoid arthritis drug tofacitinib. Specifically, the invention relates to 1-benzyl-4-methyl-5-alkoxy-1,2,3,6-tetrahydropyridine compounds, synthetic methods thereof and their application in preparing the key intermediate of tofacitinib, cis-1-benzyl-3-methylamino-4-methylpiperidine.

## Description

### Technical Field

The invention relates to the field of synthesis of drug intermediates, in particular to the field of synthesis of key intermediates for preparing anti-rheumatoid arthritis drug tofacitinib. Specifically, the invention relates to 1-benzyl-4-methyl-5-alkoxy-1,2,3,6-tetrahydropyridine compounds, synthetic methods thereof and their application in preparing the key intermediate of tofacitinib, cis-1-benzyl-3-methylamino-4-methylpiperidine.

### Background

Tofacitinib citrate is the first inhibitor of the JAK pathway developed by Pfizer for the treatment of rheumatoid arthritis. The product was first approved for marketing in the United States on November 6, 2012. Tofacitinib is generally synthesized by conversion of the key intermediate compound of formula VI, (3R,4R)-1-benzyl-3-methylamino-4-methylpiperidine (Scheme 1).

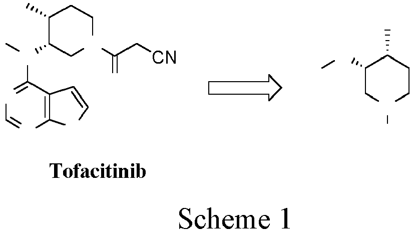

There are many reports in the literature about the synthesis of the intermediate compound of formula VI. Org. process. Res. Dev. 2003, 7, 115-120 reported a preparation method using 4-picoline as starting material, including reaction with benzyl halide, reduction, hydroboration-oxidation, further oxidation to ketone, followed by reductive amination with methylamine to obtain a racemic intermediate, which was resolved to give the product (Scheme 2). The synthetic route is not conducive to industrial production due to the long route, cumbersome operation of the hydroboration-oxidation step, and the use of dangerous and regulated hydrogen peroxide and sulfur trioxide pyridine reagents in the subsequent oxidation step.

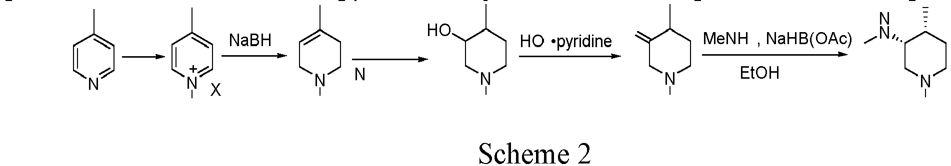

Org. process. Res. Dev. 2005, 9, 51-56 reported a preparation method using 3-amino-4-methylpyridine as starting material, which was subjected to amino protection, hydrogenation, reduction, and benzylation to obtain 1-benzyl-3-methylamino-4-methylpiperidine, which was then resolved (Scheme 3). The total yield of this route is low, only 14.6%, and expensive metal Rh/C catalyst and dangerous reductant lithium aluminum hydride are used, which limits industrial production.

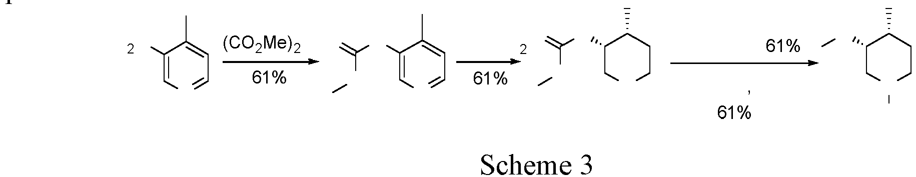

J. Med. Chem, 2008, 51, 8102-8018 reported a method for synthesizing a series of derivatives of tofacitinib (Scheme 4). However, the route of this method is lengthy and cumbersome, expensive metal catalyst platinum dioxide is used, and the obtained intermediate comprises 4 isomers, making this method not suitable for industrialization.

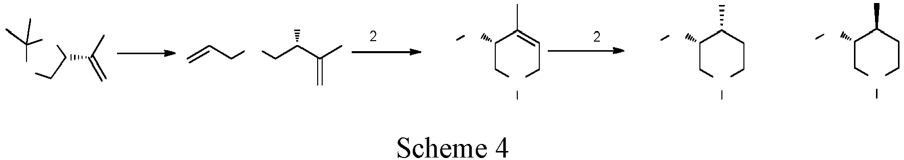

Patent application CN108610279A reported a method using 3-amino-4-methylpyridin as starting material, and a racemic intermediate is synthesized via reductive amination, salt formation and reduction in three steps (Scheme 5). The starting material of this route is expensive and difficult to obtain, and the work-up of the product obtained by reduction and acidification is relatively cumbersome.

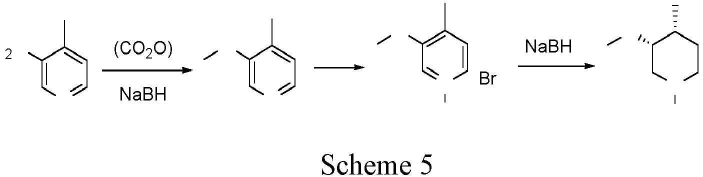

Patent application WO2014097150 and CN108689915A reported synthesis methods using 3-bromo-4-methylpyridin as starting material. Said methods utilize the Ullmann reaction to achieve aminomethylation. The difference between the two is that the former first forms a salt and then perform the aminomethylation, while the latter first undergoes aminomethylation and then forms a salt (Scheme 6). The Ullmann reaction in these routes has the disadvantages of relatively harsh reaction conditions low yield, cumbersome work-up and the like.

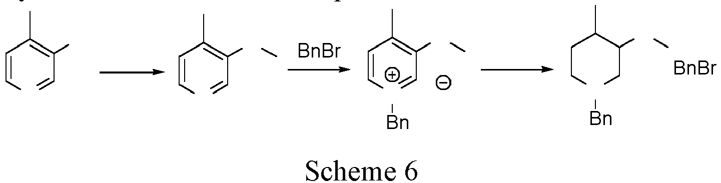

In summary, the key points of the preparation method of the compound VI reported so far are the two steps of piperidine ring construction and aminomethylation, but each method currently known has the problems of cumbersome process operations, use of regulated and dangerous reagents, use of noble metal catalysts, unsatisfactory yield, harsh reaction conditions and the like. Therefore, there is a need to further develop a simple and efficient synthesis method suitable for the industrial production of compound VI.

In order to overcome the shortcomings and deficiencies of the prior art, the invention provides a new synthesis method of compound VI. The method has the characteristics of convenient operation, cheap and readily available starting materials, high product yield, high purity of the intermediates and the target product and the like, and is easy for industrial production.

### Disclosure of Invention

In the present invention, the following terms have the meanings as described below:
The term "alkyl", alone or in combination with other groups, denotes a straight-chain or branched monovalent saturated hydrocarbon group consisting of carbon and hydrogen atoms. "C₁-C₁₀ alkyl" denotes alkyl having 1 to 10 carbon atoms, preferably C₁-C₆ alkyl. "C₁-C₆ alkyl" denotes a branched or straight chain alkyl group having 1 to 6 carbon atoms, such as methyl, ethyl, n-propyl, isopropyl, n-butyl, sec-butyl, tert-butyl, n-pentyl, isopentyl, n-hexyl.

"Halogen" means fluorine, chlorine, bromine, iodine.

"Haloalkyl" means an alkyl group as described above substituted with one or more halogens, preferably halo C₁-C₆ alkyl, for example trifluoromethyl.

"Cycloalkyl" means a cyclic saturated alkyl group consisting of carbon and hydrogen atoms, preferably C₃-C₇ cycloalkyl, such as cyclopropyl, cyclobutyl, cyclopentyl, cyclohexyl or cycloheptyl.

"Aryl" refers to a monocyclic or fused bicyclic aromatic ring consisting of carbon and hydrogen atoms. "C₅₋₁₀ aryl" refers to an aryl group containing 5 to 10 carbon atoms. For example, C₅₋₁₀ aryl can be phenyl or naphthyl.

"Aralkyl" refers to an alkyl group as described above substituted with an aryl group as described above, for example benzyl.

"Substituted alkyl", "substituted cycloalkyl", "substituted aryl" and "substituted aralkyl" refer to alkyl, cycloalkyl, aryl and aralkyl groups as described above substituted with alkyl, halogen, haloalkyl, hydroxy and/or amino groups as described above. Examples include, but are not limited to, 2-hydroxy-ethyl, 2-amino-ethyl, 3-hydroxypropyl, 4-hydroxy-butyl, methylcyclohexyl, 2-methylphenyl, 4-methylphenyl, 4-propylbenzyl, 4-methylbenzyl, and the like.

"C₁-C₄ alcohol" refers to an alcohol having 1-4 carbon atoms, such as methanol, ethanol, n-propanol, isopropanol, n-butanol, isobutanol, or t-butanol.

The invention aims to provide a synthesis method of the key intermediate for preparing tofacitinib, namely cis-1-benzyl-3-methylamino-4-methylpiperidine. The method has the advantages of short route, readily available starting materials, mild conditions, convenient purification, high yield and easy realization of large-scale production.

The technical problem to be solved by the invention is realized by the following technical solutions.

In a first aspect, the present invention provides a compound of formula I, 1-benzyl-4-methyl-5-alkoxy-1,2,3,6-tetrahydropyridine: 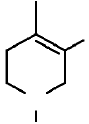 wherein R is selected from an alkyl group, a substituted alkyl group, a cycloalkyl group, a substituted cycloalkyl group, an aryl group, a substituted aryl group, an aralkyl group, or a substituted aralkyl group.

For example, R is selected from methyl, ethyl, n-propyl, isopropyl, n-butyl, isobutyl, tert-butyl, 2-hydroxy-ethyl, 2-amino-ethyl, 3-hydroxypropyl, 4-hydroxy-butyl, pentyl, cyclopentyl, hexyl, cyclohexyl, methylcyclohexyl, phenyl, 2-methylphenyl, p-methylphenyl, benzyl, 4-propylbenzyl or 4-methylbenzyl.

In a preferred embodiment, R is selected from C₁-C₆ alkyl, cyclohexyl, 2-hydroxyethyl, 2-aminoethyl, 3-hydroxypropyl, 4-hydroxybutyl, benzyl or phenyl.

More preferably, R is selected from methyl, ethyl, n-propyl, isopropyl, n-butyl, isobutyl, cyclohexyl, 2-hydroxyethyl, 2-aminoethyl, 3-hydroxypropyl or benzyl.

In a second aspect, the present invention provides a method for the preparation of a compound of formula I, 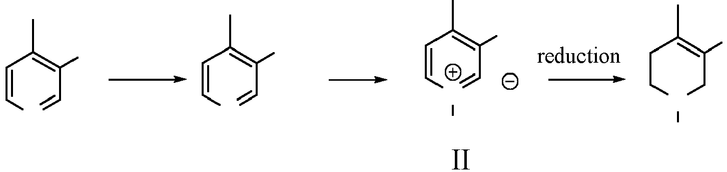 wherein L is a leaving group, R is as described above for the compound of formula I, M is a metal element, n is selected from 1, 2 or 3, and X is halogen, said comprises the steps of:
step (1): reacting the compound of formula IV with ROH or (RO)ₙM to obtain the compound of formula III,
step (2): reacting the compound of formula III with benzyl halide to obtain the compound of formula II, and
step (3): reducing the compound of formula II under the action of a reductant to obtain the compound of formula I.

In some preferred embodiments, the specific reaction conditions are as follows:
Step (1): under an inert gas atmosphere, in a polar solvent, the compound of formula IV undergoes a substitution reaction with ROH or (RO)ₙM under the catalysis of copper ions catalyst or palladium catalyst to obtain the compound of formula III.

The copper ion catalyst is selected from cuprous chloride, cupric chloride, cuprous bromide, cupric bromide, cuprous iodide, cupric iodide, cupric sulfate, cupric nitrate, cupric acetate, cupric hydroxide or cupric acetylacetonate.

The palladium ion catalyst is selected from palladium chloride, triphenylphosphine palladium chloride and palladium acetate.

In the compound of formula IV, L is halogen such as F, Cl, Br or I, or L is -OCOR¹ , -OR² or -ONO₂.

R¹ is alkyl or haloalkyl, for example, CF₃ or CH₃.

R² is alkylsulfonyl or arylsulfonyl, or substituted alkylsulfonyl or arylsulfonyl, such as p-toluenesulfonyl, p-bromobenzenesulfonyl, nitrobenzenesulfonyl, trifluoromethanesulfonyl, methanesulfonyl, 5-(dimethylamino)naphthalene-1-sulfonyl.

M in (RO)ₙM is a metal element, and M can be selected from lithium, sodium, potassium, calcium, magnesium, aluminum, zinc or iron, n is the valence of the metal M, and n is selected from 1, 2 or 3.

In some specific embodiments, when M is lithium, sodium, potassium, n is 1; when M is calcium, magnesium or zinc, n = 2; when M is aluminum or iron, n = 3.

In a more preferred embodiment, L is Cl or Br.

The R substituent in (RO)ₙM is as described above.

Preferably, said (RO)ₙM is a solid alkali metal salt or a solution thereof.

For example, the alkali metal salt is a lithium salt, a sodium salt or a potassium salt, and the solution of the alkali metal salt includes a solution of the lithium salt, the sodium salt or the potassium salt in C₁-C₄ alcohol. Alternatively, the (RO)ₙM solution can be prepared in situ by adding a strong base to the ROH.

In the method for preparing the (RO)ₙM solution in situ, the strong base is selected from one or more of metallic lithium, metallic sodium, metallic potassium, sodium hydride, potassium hydride, calcium hydride, sodium tert-butoxide, potassium tert-butoxide, sodium hydroxide, potassium hydroxide or Grignard reagents.

The Grignard reagent is an alkyl magnesium halide or an aryl magnesium halide, such as methyl magnesium bromide, ethyl magnesium bromide, ethyl magnesium iodide, propyl magnesium bromide, butyl magnesium bromide, phenyl magnesium bromide.

The feeding ratio of the compound of formula IV to ROH is 1.0: 1.0-20.0.

The feeding ratio of the compound of formula IV to (RO)ₙM is 1.0: (1.0-20.0)/n, and n is selected from 1, 2 or 3.

The copper ion is a monovalent or divalent copper ion, including but not limited to cuprous chloride, cuprous bromide, cuprous iodide, cupric chloride, cupric bromide, cupric iodide, cupric sulfate, cupric nitrate, copper acetate.

The amount of the catalyst used is 0.1 to 20.0 mol%, preferably 0.5 to 10.0 mol%.

The reaction solvent is a polar aprotic solvent, a protic solvent or a mixture thereof, including N,N-dimethylformamide, N,N-dimethylacetamide, dimethylsulfoxide, N-methylmorpholine, methanol, ethanol, n-propanol, isopropanol, n-butanol, isobutanol, ethylene glycol, propylene glycol, butylene glycol, or a mixture of any two or more of them.

The reaction temperature is 20-150 °C, for example 60-150 °C, preferably 100-130 °C.

The reaction time is 1.0-10.0 hours.

Step (2): reacting the compound of formula III with benzyl halide to obtain the compound of formula II.

The benzyl halide may be benzyl chloride, benzyl bromide or benzyl iodide. The feeding ratio of the compound of formula III to the benzyl halide is 1: 1.0-3.0.

The reaction temperature is 0 to 150 °C, preferably 10 to 80 °C.

The reaction time is 2-48 h.

Step (3): reducing the compound of formula II in a protic solvent with a reductant to obtain the compound of formula I.

The protic solvent used may be methanol, ethanol, n-propanol, isopropanol, n-butanol, isobutanol, ethylene glycol, propylene glycol or a mixture thereof.

The reductant used may be a reductant commonly used in the art, such as lithium borohydride, sodium borohydride, potassium borohydride, sodium cyanoborohydride, lithium triethylborohydride or borane.

The reaction temperature is -5- 30 °C.

The reaction time is 0.5-24 h.

In a third aspect, the present invention also provides a synthetic method of the key intermediate for preparing tofacitinib, which is a compound of formula VI (i.e., cis-1-benzyl-3-methylamino-4-methylpiperidine), comprising the steps of: 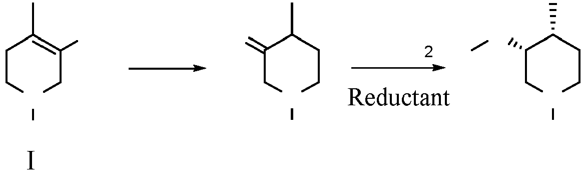
step (4): hydrolyzing the compound of formula I under acidic conditions to obtain the compound of formula V, and
step (5): reductive amination of the compound of formula V with methylamine in the presence of a reductant, and salifying with hydrochloric acid to obtain the compound of formula VI.

In some preferred embodiments, the specific reaction conditions are as follows: Step (4): hydrolyzing the compound 1-benzyl-4-methyl-5-alkoxy-1,2,3,6-tetrahydropyridine of the formula I under acidic conditions to obtain the compound 1-benzyl-4-methylpiperidine-3-one of the formula V.

The acid used in the reaction is an inorganic acid or an organic acid. For example, the inorganic acid may include hydrochloric acid, hydrobromic acid, hydroiodic acid, sulfuric acid, phosphoric acid, nitric acid, hypochlorous acid, hypobromous acid, hypophosphorous acid, hyposulfuric acid. The organic acid may include formic acid, acetic acid, trifluoroacetic acid, oxalic acid, tartaric acid, citric acid, p-toluenesulfonic acid, trifluoromethanesulfonic acid.

The acid used for the reaction is preferably hydrochloric acid, for example, 6M hydrochloric acid solution.

The feeding ratio of the compound of formula I to the acid is 1: 1-1: 10.

The reaction solvent is a protic or an aprotic organic solvent, such as methanol, ethanol, n-propanol, isopropanol, dichloromethane, tetrahydrofuran, 2-methyltetrahydrofuran, 1,4-dioxane, and toluene, and the reaction temperature is 0-110 °C, and the reaction time is 0.5-10 hours.

Step (5): reacting the compound of formula V with methylamine to form Schiff base, then reacting with a reductant to obtain the compound of formula VI, namely 1-benzyl-3-methylamino-4-methylpiperidine, and salifying the compound of formula VI with hydrochloric acid for further purification.

The methylamine used is an acid addition salt of methylamine or an alcoholic solution of methylamine. The acid addition salt of methylamine is a hydrochloride, hydrobromide, hydroiodide, sulphate, formate or acetate of methylamine.

The reductant used is a reductant commonly used in the art, such as sodium cyanoborohydride, sodium triacetoxyborohydride, lithium triethylborohydride, borane, (S)-2-methyl-CBS-oxazaborolidine, or the reductant can be prepared in situ according to a conventional method, for example, sodium triacetoxyborohydride can be prepared according to the method described in Org. process. Res. Dev. 2003, 7, 115-120.

In a preferred embodiment, the method for preparing the compound of formula VI comprises the following steps: 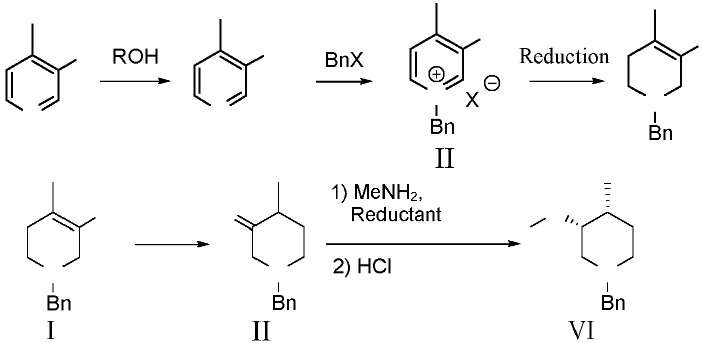
step (1): reacting the compound of formula IV with ROH or (RO)ₙM to obtain the compound of formula III,
step (2): reacting the compound of formula III with benzyl halide to obtain the compound of formula II,
step (3): reducing the compound of formula II under the action of a reductant to obtain the compound of formula I,
step (4): hydrolyzing the compound of formula I under acidic conditions to obtain the compound of formula V, and
step (5): reductive amination of the compound of formula V with methylamine in the presence of a reductant, and salifying with hydrochloric acid to obtain the compound of formula VI,
wherein L is a leaving group, R is as defined in claim 1, M and n are as defined in claim 3, and X is halogen.

In a further preferred embodiment, the reaction conditions for each of steps (1), (2), (3), (4), (5) are as described above.

### Detailed Description

The process of the present invention is further illustrated by the following examples. It should be understood that the following examples are provided only for the purpose of enabling a better understanding of the present invention, and are not intended to limit the scope of the present invention in any way.

Examples 1-4 prepare 1-benzyl-5-methoxy-4-methyl-1,2,3,6-tetrahydropyridine through the following route.

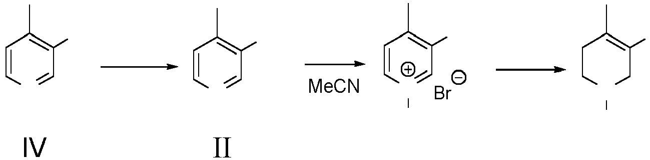

### Example 1

### Preparation of 3-methoxy-4-methylpyridine:

Under the protection of nitrogen, 17.2 g of 3-bromo-4-methylpyridine and 100.0 mL of DMF were added to a 250 mL three-necked flask and dissolved, followed by addition of 10.8 g of sodium methoxide and 0.72 g of CuBr, and the reaction mixture was heated to 130 ° C and stirred for 5 hours. TLC (PE: EA = 2:1) monitored the complete reaction of the starting materials, and white solid NaBr was gradually formed. The reaction mixture was cooled to room temperature, and 40.0 mL of water was added to quench the reaction. The mixture was extracted by adding methyl tert-butyl ether (60 mL × 3), the organic layers were combined, washed by adding 20.0 mL of saturated NaCl, and concentrated by rotary evaporation to obtain 17.50 g of a dark yellow liquid with a crude yield of 100% and a GC purity of 91.5%, which was directly used for the next reaction without purification. ¹H-NMR (400 MHz, CDCl₃) δ 8.16 (s, 1H), 8.12 (d, *J* = 4.8 Hz, 1H), 7.06 (d, *J* = 4.8 Hz, 1H), 3.91 (s, 3H), 2.23 (s, 3H).

### Example 2

### Preparation of 1-benzyl-3-methoxy-4-methylpyridinium chloride:

Under the protection of nitrogen, 17.5 g of the above reaction product 3-methoxy-4-methylpyridine was added into a 250 mL reaction flask, dissolved in 120 mL acetonitrile, added with 13.8 mL benzyl chloride, heated to 80 ° C and stirred under reflux for 7 hours. TLC (PE: EA = 2:1) showed that the starting materials were reacted completely and the color of the solution gradually became darker. The reaction mixture was cooled to room temperature, the reaction solution was evaporated to dryness by rotation, petroleum ether was added, stirred and dispersed at room temperature. The petroleum ether was removed by suction filtration, and the filter cake was sucked to dryness to obtain 22.6 g of off-white solid with a crude yield of 90.4%. The product was directly used for the next reaction without purification.

### Example 3

### Preparation of 1-benzyl-5-methoxy-4-methyl-1,2,3,6-tetrahydropyridine:

22.6 g of 1-benzyl-3-methoxy-4-methylpyridinium chloride obtained above was charged into a 500 mL reaction flask, dissolved in 220 mL of methanol, and the internal temperature was controlled at 10 - 25 °C, and 10.26 g of NaBH₄ was added in portions. The reaction was violently exothermic and accompanied by gas evolution. After the addition was complete, stirring was continued for 2 hrs at room temperature, and the reaction was monitored by TLC (DCM: MeOH = 10:1) for completion. The reaction was quenched by adding 80 mL of water, methanol was evaporated, EtOAc was added (100mL×2), the organic layers were combined, washed with water and distilled to dryness to give 17.0 g of a light yellow liquid with a yield of 87.0%. ¹H-NMR (400 MHz, CDCl₃) δ 7.36-7.25 (m, 5H), 3.59 (s, 2H), 3.48 (s, 3H), 2.98-2.96 (m, 2H), 2.50 (t, *J* = 5.6 Hz, 2H), 2.07-2.05 (m, 2H), 1.63 (s, 3H); ¹³C-NMR (100 MHz, CDCl₃) δ 145.8, 137.9, 129.2, 128.2, 127.1, 113.3, 62.6, 57.4, 51.7, 49.8, 30.0, 14.9 ppm.; MS (EI): 217.2, (C₁₄H₁₉NO [M]⁺).

### Example 4

### Preparation of 1-benzyl-5-methoxy-4-methyl-1,2,3,6-tetrahydropyridine:

2.70 g of the above-mentioned 1-benzyl-3-methoxy-4-methylpyridinium chloride was charged into a 50 mL reaction flask dissolved in 20 mL of methanol, 1.05 g of NaBH₄ was added in portions while controlling the internal temperature at 10 - 25 °C. The reaction was exothermic with gas evolution. After the addition was complete, stirring was continued for 2 hrs at room temperature, and the reaction was monitored by TLC (v/v DCM: MeOH = 10:1) for completion. The reaction was quenched by adding 10 mL of water, methanol was evaporated, extracted with EtOAc (10 mL ×2), the organic layers were combined, washed with water and distilled to dryness to give 1.64 g of light yellow liquid with a yield 82.1%.

### Examples 5-7 synthesize 1-benzyl-5-ethoxy-4-methyl-1,2,3,6-tetrahydropyridine through the following route:

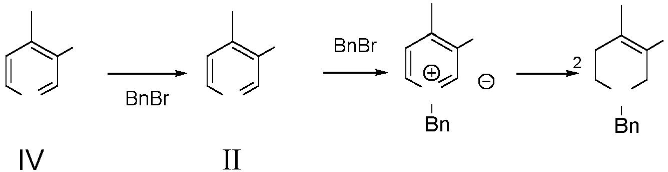

### Example 5

### Preparation of 3-ethoxy-4-methylpyridine:

Under the protection of nitrogen, 17.2 g of 3-bromo-4-methylpyridine and 100 mL of DMF were added to a 250 mL three-necked flask and dissolved, followed by addition of 13.6 g of sodium ethoxide and 0.67 g of CuCl₂, and the reaction mixture was heated to 130 ° C and stirred for 7 hours. TLC (v/v PE: EA = 2:1) monitored the complete reaction of the starting materials, and white solid NaBr was gradually formed. The reaction mixture was cooled to room temperature, and 40 mL of water was added to quench the reaction. The mixture was extracted by adding methyl tert-butyl ether (60 mL × 3), the organic layers were combined, washed with water, and concentrated to dryness to obtain 11.20 g of dark yellow liquid with a crude yield of 81.8%. ¹H-NMR (400 MHz, CDCl₃) δ 8.15 (s, 1H), 8.10 (d, *J* = 4.0 Hz, 1H), 7.05 (d, *J* = 4.4 Hz, 1H), 4.12 (q, *J* = 6.8 Hz, 2H), 2.23 (s, 3H), 1.44 (t, *J* = 7.2 Hz, 3H).

### Example 6

### Preparation of 1-benzyl-3-ethoxy-4-methylpyridinium chloride:

Under the protection of nitrogen, 11.2 g (82 mmol) of the above reaction product 3-ethoxy-4-methylpyridine was added into a 250 mL reaction flask and dissolved in 120 mL acetonitrile, added with 12.4 g benzyl chloride, heated to 80 ° C and stirred under reflux for 7 hours. TLC (v/v PE: EA = 2:1) showed that the starting materials were reacted completely and the color of the solution gradually became darker. The reaction mixture was cooled to room temperature, the reaction solution was evaporated to dryness, petroleum ether was added, stirred and dispersed at room temperature. The petroleum ether was removed by suction filtration, and the filter cake was sucked to dryness to obtain 18.3 g of off-white solid with a crude yield of 98.0%. The product was directly used for the next reaction without purification.

### Example 7

### Preparation of 1-benzyl-5-ethoxy-4-methyl-1,2,3,6-tetrahydropyridine:

18.3 g of 1-benzyl-3-ethoxy-4-methylpyridinium chloride obtained above was charged into a 500 mL reaction flask and dissolved in 200 mL of ethanol, and the internal temperature was controlled at 10 - 25 °C, and 8.78 g of NaBH₄ was added in portions. The reaction was slowly, exothermic with gas evolution. After the addition was complete, stirring was continued for 2 hrs at room temperature, and the reaction was monitored by TLC (DCM: MeOH = 10:1) for completion. The reaction was quenched by adding 80 mL of water, ethanol was evaporated, extracted with EtOAc (100mL×2), the organic layers were combined, washed with water and distilled to dryness to give 16.7 g of a light yellow liquid with a yield of 90.0%. ¹H-NMR (400 MHz, CDCl₃) δ 7.35-7.24 (m, 5H), 3.66 (q, *J* = 6.8 Hz, 2H), 3.58 (s, 2H), 2.96 (s, 2H), 2.50 (t, *J* = 4.2 Hz, 2H), 2.06 (m, 2H), 1.63 (s, 3H), 1.21 (t, *J* = 7.2 Hz, 3H); ¹³C-NMR (100 MHz, CDCl₃) δ 144.8, 138.2, 129.2, 128.2, 127.1, 113.7, 65.1, 62.6, 52.5, 49.9, 30.1, 15.6, 15.2 ppm.; MS (EI): 231.1, (C₁₅H₂₁NO [M ]⁺).

### Examples 8-10 synthesize 1-benzyl-5-isopropoxy-4-methyl-1,2,3,6-tetrahydropyridine through the following route:

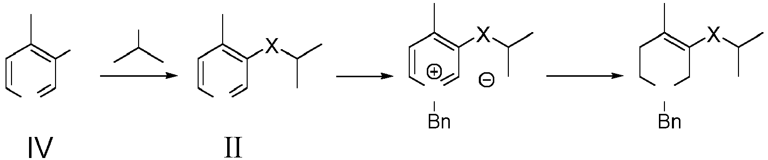

### Example 8

### Preparation of 3-isopropoxy-4-methylpyridine:

Under the protection of nitrogen, 20 mL of isopropanol was added into a 250 mL three-necked flask, NaH 2.0 g (60%) was added in portions while controlling the temperature at -5 to 5 °C to prepare sodium isopropoxide, and then 40 mL of DMF, 4.3 g of reaction substrate 3-bromo-4-methylpyridine and 180 mg of CuBr were added in sequence, and the system turned light blue. The reaction was heated to 130 °C and stirred for 4 hours, and TLC (PE: EA = 2: 1) monitoring showed that the reaction of the raw material was substantially complete. The reaction mixture was cooled to room temperature, and 20 mL of water was added to quench the reaction. The remaining isopropanol was distilled off under reduced pressure, and the remaining liquid was extracted by adding methyl tert-butyl ether (20 mL×3). Floccules were formed, the mixture was filtered, the organic phases were combined, washed with water, and concentrated to dryness to obtain 2.4 g of a light yellow liquid. Yield: 63.6%. The product was directly used for the next reaction without purification. ¹H-NMR (400 MHz, CDCl₃) δ 8.18 (s, 1H), 8.09 (d, *J* = 4.4 Hz, 1H), 7.06 (d, *J* = 4.4 Hz, 1H), 4.63-4.57 (m, 1H), 2.22 (s, 3H), 1.36 (d, *J* = 6.0 Hz, 6H).

### Example 9

### Preparation of 1-benzyl-3-isopropoxy-4-methylpyridinium chloride:

Under the protection of nitrogen, 2.4 g of the above reaction product 3-isopropoxy-4-methylpyridine and 30 mL of acetonitrile were added to a 100 mL reaction flask, and after dissolving, 2.43 g of benzyl chloride was added, the mixture was heated to 80 ° C and stirred under reflux for 5 hours, and the completion of reaction of the starting materials was monitored by TLC (PE: EA = 2: 1). The reaction mixture was cooled to room temperature, the reaction solution was concentrated to dryness, petroleum ether was added, stirred and dispersed at room temperature. The petroleum ether was removed by suction filtration, and the filter cake was sucked to dryness to obtain 4.0 g of white solid with a crude yield of 90.0%. The product was used for the next reaction without purification.

### Example 10

### Preparation of 1-benzyl-5-isopropoxy-4-methyl-1,2,3,6-tetrahydropyridine

4.0 g of the above-mentioned 1-benzyl-3-isopropoxy-4-methylpyridinium chloride and 40 mL of methanol were added to a 100 mL reaction flask and dissolved, the internal temperature was controlled at 10 - 25°C, and 1.64 g of NaBH₄ was added in portions. The reaction was exothermic with gas evolution. After the addition was complete, stirring was continued for 1 hrs at room temperature, and the reaction was monitored by TLC (DCM: MeOH = 10:1) for completion. The reaction was quenched by adding 20 mL of water, methanol was evaporated, EtOAc was added for extraction (40mL×2), the organic layers were combined, washed with water and concentrated to dryness to give 2.97 g of product as a colorless liquid in 84.2% yield. ¹H-NMR (400 MHz, CDCl₃) δ 7.34-7.22 (m, 5H), 3.98-3.92 (m, 1H), 3.57 (s, 2H), 2.92 (s, 2H), 2.50 (t, *J* = 4.2 Hz, 2H), 2.07 (s, 2H), 1.62 (s, 3H), 1.15 (d, *J* = 6.0 Hz, 6H); ¹³C-NMR (100 MHz, CDCl₃) δ 143.3, 138.3, 129.1, 128.2, 127.0, 114.2, 70.3, 62.6, 53.2, 50.0, 30.2, 22.7, 15.7 ppm.; MS (EI): 245.2, (C₁₆H₂₃NO [M ]⁺).

### Examples 11-13 synthesize 1-benzyl-5-(2-hydroxy-ethoxy)-4-methyl-1,2,3,6-tetrahydropyridine through the following route:

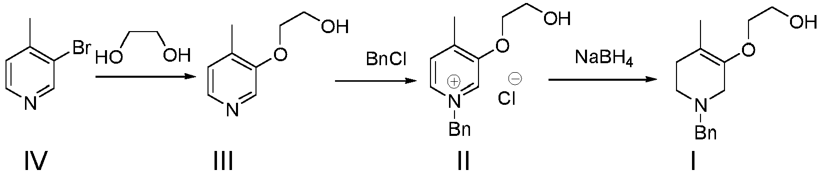

### Example 11

### Preparation of 3-(2-hydroxy-ethoxy)-4-methylpyridine:

Under the protection of nitrogen, 12.4 g of ethylene glycol, 3.44 g of 3-bromo-4-methylpyridine, 1.6 g of NaOH, and 134 mg of CuCl₂ were added to a 100 mL three-necked flask, and after mixing, the system turned dark blue. The reaction was heated to 130 ° C and stirred for 3 hours, and TLC (v/v DCM: MeOH = 10: 1) monitoring showed the substantial completion of the reaction. The reaction mixture was cooled to room temperature, distilled under reduced pressure to remove ethylene glycol, and extracted with dichloromethane (40 ml ×2). The organic phases were combined, washed with water, concentrated to dryness, and n-hexane was added for crystallization. The precipitate was filtered to obtain 2.14 g of the product as a light yellow solid. Yield: 70.0%. ¹H-NMR (400 MHz, CDCl₃) δ 8.12 (s, 1H), 8.08 (d, *J* = 4.4Hz,1H),7.04 (d, *J* = 4.4Hz, 1H), 4.15-4.13 (t, 2H), 3.99-3.96 (t, 2H), 2.22 (s, 3H).

### Example 12

### Preparation of 1-benzyl-3- (2-hydroxy-ethoxy)-4-methylpyridinium chloride:

To a 100 mL reaction flask were added 2.14 g of 3-(2-hydroxy-ethoxy)-4-methylpyridine, the product of the previous step, and 25 mL of acetonitrile. 2.12 g of benzyl chloride was added, the mixture was heated and stirred at reflux for 6 hours, and the completion of reaction of the starting materials was monitored by TLC (DCM: MeOH = 10: 1). The reaction mixture was cooled to room temperature, and a solid precipitated out. Acetonitrile was removed by concentration, and 30 mL of methyl tert-butyl ether was added for dispersion. After suction filtration, 3.7 g of an off-white solid was obtained. Yield: 94.0%.

### Example 13

### Preparation of 1-benzyl-5-(2-hydroxy-ethoxy)-4-methyl-1,2,3,6-tetrahydropyridine

3.65 g of 1-benzyl-3-(2-hydroxy-ethoxy)-4-methylpyridinium chloride was added to a 100 mL reaction flask and dissolved in 50 mL methanol. The mixture was cooled to 10 ° C, and 1.25 g of NaBH₄ was added in portions. After the addition was complete, the mixture was warmed to room temperature and stirred for 3h, and TLC (v/v DCM: MeOH = 10: 1) monitoring showed substantial completion of the reaction. The reaction was quenched by the addition of 10 mL of water, methanol was removed by rotary evaporation, the residue was extracted by the addition of ethyl acetate (20 mL ×2), washed with water, and concentrated to dryness to give 2.75 g of a light yellow liquid in 85% yield. ¹H-NMR (400 MHz, CDCl₃) δ 7.42-7.27 (m, 5H), 3.75-3.74 (m, 6H), 3.14 (m, 2H), 2.64-2.66 (m, 2H), 2.16 (m, 2H), 2.16 (s, 3H); ¹³C-NMR (100 MHz, CDCl₃) δ 143.6, 135.7, 129.7, 128.6, 127.9, 114.6, 71.3, 62.0, 61.8, 51.6, 49.3, 29.2, 15.2 ppm; MS (ESI): 248.05, (C₁₅H₂₁NO₂, [M+1]⁺).

### Examples 14-17 illustrate synthetic procedures for preparing intermediate compound VI from the compound of formula I

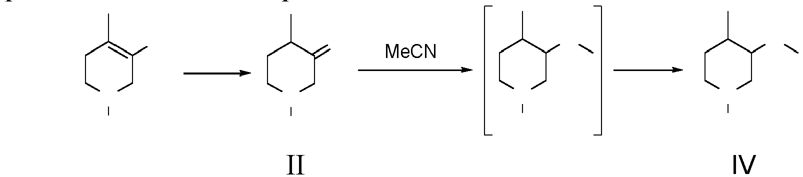

### Example 14

### Preparation of 1-benzyl-4-methylpiperidine-3-one (Compound V), Method I:

To a 250 mL reaction flask was added 17.0 g of 1-benzyl-5-methoxy-4-methyl-1,2,3,6-tetrahydropyridine and 150 mL of methanol, after dissolving, 39 mL of 6M HCl was added, the reaction was heated to 60 ° C and refluxed for 2h, and TLC (v/v PE: EA = 2: 1) monitoring showed complete conversion of the starting material. The reaction mixture was cooled to room temperature, methanol was evaporated under reduced pressure, 50 mL of water was added, and NaOH solid was added to adjust the pH value to be greater than 11, ethyl acetate (50 mL ×2) was added for extraction. The organic layer was washed with water, and rotary evaporated to dryness to obtain 21.0 g of a reddish brown crude product (crude yield: 100%), which was directly used for the next reaction without purification. A small amount of crude product was subjected to column chromatography to obtain a light yellow liquid, which was used for structure identification. ¹H-NMR (400 MHz, CDCl₃) δ 7.34-7.26 (m, 5H), 3.58-3.57 (m, 2H), 3.25-3.21(d, *J* = 14.0 Hz, 1H), 2.95-2.92 (m, 1H), 2.80-2.77 (d, *J* = 14.0 Hz, 1H), 2.47-2.45 (m, 1H), 2.36-2.33 (m, 1H), 2.06-2.01 (m, 1H), 1.66-1.63 (m, 1H), 1.08 (d, *J* = 6.4 Hz, 3H); ¹³C-NMR (100 MHz, CDCl₃) δ 208.6, 137.2, 129.2, 128.4, 127.5, 64.1, 62.6, 51.9, 43.0, 32.9., 14.2 ppm; ESI-MS: 204.05, (C₁₃H₁₇NO, [M+1]⁺).

### Example 15

### Preparation of 1-benzyl-4-methylpiperidine-3-one (Compound V), Method II:

2.0 g of 1-benzyl-5-ethoxy-4-methyl-1,2,3,6-tetrahydropyridine and 20 mL of MeOH were added to a 100 mL reaction flask, after dissolving, 4.8 mL of 6M HCl was added, the reaction was heated to 60° C and refluxed for 2h, and TLC (v/v PE: EA = 2: 1) monitoring showed complete conversion of the starting material. The reaction mixture was cooled to room temperature, methanol was evaporated under reduced pressure, 20 mL of water was added, and NaOH solid was added to adjust the pH value to be greater than 11, ethyl acetate (15 mL ×2) was added for extraction. The organic layer was washed with water, and rotary evaporated to dryness to obtain 2.1 g of a dark yellow crude product (crude yield: 100%), which was directly used for the next reaction.

### Example 16

### Preparation of 1-benzyl-4-methylpiperidine-3-one (Compound V), Method III:

2.66 g of 1-benzyl-5-(2-hydroxy-ethoxy)-4-methyl-1,2,3,6-tetrahydropyridine and 20 mL of methanol were added to a 100 mL reaction flask, after dissolving, 5 mL of 6M HCl was added, the reaction was heated to 60 ° C and refluxed for 2h, and TLC (v/v PE: EA = 2: 1) monitoring showed complete conversion of the starting material. The reaction mixture was cooled to room temperature, methanol was evaporated under reduced pressure, 20 mL of water was added, and NaOH solid was added to adjust the pH value to be greater than 11, ethyl acetate (15 mL ×2) was added for extraction. The organic layer was washed with water, and rotary evaporated to dryness to obtain 2.13 g of a dark yellow crude product (crude yield: 100%), which was directly used for the next reaction.

### Example 17

### Preparation of 1-benzyl-3-methylamino-4-methylpiperidine hydrochloride:

Under the protection of nitrogen, 21.0 g of 1-benzyl-4-methylpiperidine-3-one and 60 mL of toluene were added to a 250 mL reaction flask, after dissolving, the temperature was controlled at 15 °C, and 50 mL of 30% methylamine in ethanol was added dropwise, and then 4.8 mL of AcOH was added. After the addition was finished, the mixture was stirred at room temperature for 2 hours to prepare an imine intermediate, and then the imine intermediate was poured into a dropping funnel.

To a 500 mL three-necked flask, 3.95 g of NaBH₄ was added and dissolved in 80 mL THF. The mixture was cooled to 10 °C, and 28.3 g of acetic acid was slowly added dropwise. The reaction was violently exothermic and accompanied by gas evolution. After the addition was complete, stirring was continued for 2 hrs at 15 °C to prepare NaBH(OAc)₃ in situ. The internal temperature was controlled to be lower than 20 °C, and the imine solution prepared above was added dropwise to the system. After completion of the dropwise addition, the system was allowed to naturally warm to room temperature, and the completion of reaction was monitored by TLC (v/v PE: EA = 2: 1). Adding 30% NaOH aqueous solution to adjust the pH to 11-12, the layers were separated, the aqueous layer was extracted with 50 mL of toluene for 2 times. The organic layers were combined and concentrated to dryness under reduced pressure to obtain the crude free base. The crude product was dissolved in 100 mL EtOH, and 14 mL of concentrated hydrochloric acid was slowly added dropwise at room temperature. After the dropwise addition, the mixture was stirred for 30 min, and solids gradually precipitated out. The internal temperature was controlled at 5-10 °C and the stirring was continued slowly for 1 h. After suction filtration, the obtained pink white solid was added with 80 mL of EtOAc, slurried at room temperature, and filtered to obtain 17.8 g of white solid. The total yield of the two steps was 80.4%. Analysis of the free base, GC content 100%, dr value: 99.2: 0.8. ¹H-NMR (400 MHz, CDCl₃) δ 7.28-7.18 (m, 5H), 3.53 (d, *J* = 12.8 Hz, 1H), 3.39 (d, *J* = 12.8 Hz, 1H), 2.69-2.62 (m, 2H), 2.40 (s, 1H), 2.29 (s, 3H), 2.12-2.02 (m, 2H), 1.66 (s, 1H), 1.47-1.43 (m, 3H), 0.92 (d, *J* = 7.2 Hz, 3H); ¹³C-NMR (100 MHz, CDCl₃) δ 138.8, 128.9, 128.1, 126.8, 63.1, 59.1, 54.5, 52.6, 34.5, 33.2, 29.8, 16.3 ppm; MS (EI): 218.2, (C₁₄H₂₂N₂ [M ]⁺).

## Claims

1. A compound of formula I: 1-benzyl-4-methyl-5-alkoxy-1,2,3,6-tetrahydropyridine, 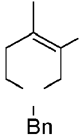 wherein R is selected from an alkyl group, a substituted alkyl group, a cycloalkyl group, a substituted cycloalkyl group, an aryl group, a substituted aryl group, an aralkyl group, or a substituted aralkyl group.

2. The compound according to claim 1, wherein R is methyl, ethyl, n-propyl, isopropyl, n-butyl, isobutyl, tert-butyl, 2-hydroxy-ethyl, 2-amino-ethyl, 3-hydroxypropyl, 4-hydroxy-butyl, pentyl, cyclopentyl, hexyl, cyclohexyl, methylcyclohexyl, phenyl, 2-methylphenyl, p-methylphenyl, benzyl, 4-propylbenzyl or 4-methylbenzyl.

3. Method for the preparation of a compound of formula (I) comprising the following three steps: 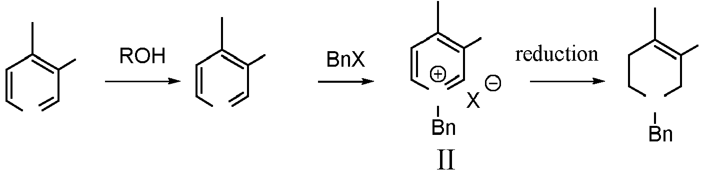 wherein L is a leaving group, R is as defined in claim 1, M is a metal element, n is selected from 1, 2 or 3, and X is halogen,
step (1): reacting the compound of formula IV with ROH or (RO)ₙM to obtain the compound of formula III,
step (2): reacting the compound of formula III with benzyl halide to obtain the compound of formula II, and
step (3): reducing the compound of formula II under the action of a reductant to obtain the compound of formula I.

4. The method according to claim 3, **characterized in that**:
L is halogen, such as F, Cl, Br or I, or L is -OCOR¹, -OR² or -ONO₂,
R¹ is alkyl or haloalkyl, e.g., CF₃ or CH₃, and/or
R² is alkylsulfonyl or arylsulfonyl, or substituted alkylsulfonyl or arylsulfonyl, such as p-toluenesulfonyl, p-bromobenzenesulfonyl, nitrobenzenesulfonyl, trifluoromethanesulfonyl, methanesulfonyl or 5-(dimethylamino)naphthalene-1-sulfonyl.

5. The method according to any one of claims 3 or 4, wherein step (1) is carried out under catalysis of a copper ion or palladium catalyst.

6. The method according to any one of claims 3 to 5, wherein M in (RO)ₙM is selected from lithium, sodium, potassium, calcium, magnesium, aluminum, zinc, or iron.

7. The method according to claim 6, wherein said (RO)ₙM is a solid alkali metal salt or a solution thereof, such as a lithium salt, a sodium salt, a potassium salt or a solution thereof in C₁-C₄ alcohol, or said (RO)ₙM solution is prepared in situ by adding a strong base to react with ROH.

8. The method according to claim 7, wherein said (RO)ₙM solution is prepared in situ by adding a strong base to react with ROH, wherein said strong base is one or more selected from the group consisting of metallic lithium, metallic sodium, metallic potassium, sodium hydride, potassium hydride, calcium hydride, sodium tert-butoxide, potassium tert-butoxide, sodium hydroxide, potassium hydroxide and Grignard reagents.

9. The method according to claim 8, wherein the Grignard reagent is an alkyl magnesium halide or an aryl magnesium halide, such as methyl magnesium bromide, ethyl magnesium bromide, ethyl magnesium iodide, propyl magnesium bromide, butyl magnesium bromide, or phenyl magnesium bromide.

10. The method according to claim 3, **characterized in that** the reaction solvent in step (1) is a polar aprotic solvent, a protic solvent or a mixture thereof, such as N,N-dimethylformamide, N,N-dimethylacetamide, dimethylsulfoxide, N-methylmorpholine, methanol, ethanol, n-propanol, isopropanol, n-butanol, isobutanol, ethylene glycol, propylene glycol, butylene glycol, or a mixture of any two or more of them.

11. The method according to claim 3, wherein in step (2), X is chlorine, bromine or iodine.

12. The method according to claim 3, wherein the reductant in step (3) is selected from lithium borohydride, sodium borohydride, potassium borohydride, sodium cyanoborohydride, lithium triethylborohydride or borane.

13. Method for the preparation of a compound of formula VI, comprising the following two steps: 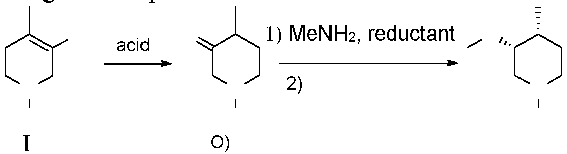
step (4): hydrolyzing the compound of formula I under acidic conditions to obtain the compound of formula V, and
step (5): reductive amination of the compound of formula V with methylamine in the presence of a reductant, and salifying with hydrochloric acid to obtain the compound of formula VI.

14. The method according to claim 13, wherein the acid used in step (4) is an organic or inorganic acid, such as hydrochloric acid, hydrobromic acid, hydroiodic acid, sulfuric acid, phosphoric acid, nitric acid, formic acid, acetic acid, trifluoroacetic acid, oxalic acid, tartaric acid, citric acid, p-toluenesulfonic acid or trifluoromethanesulfonic acid.

15. The method according to claim 13 or 14, wherein the reductant used in step (5) is sodium cyanoborohydride, sodium triacetoxyborohydride, lithium triethylborohydride, borane, (S)-2-methyl-CBS-oxazaborolidine, or the above-mentioned reductant prepared in situ according to a conventional method.

16. The method according to any one of claims 13-15, comprising the steps of: 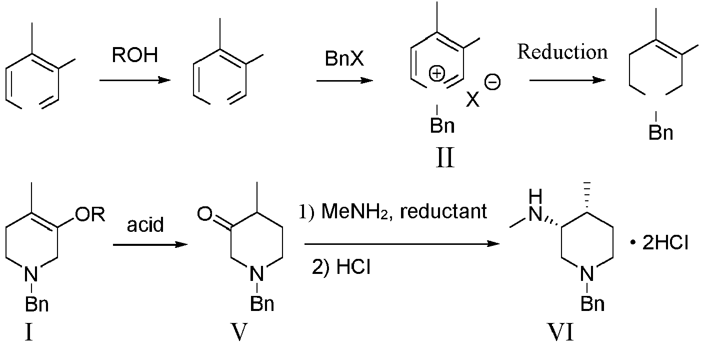
step (1): reacting the compound of formula IV with ROH or (RO)ₙM to obtain the compound of formula III,
step (2): reacting the compound of formula III with benzyl halide to obtain the compound of formula II,
step (3): reducing the compound of formula II under the action of a reductant to obtain the compound of formula I,
step (4): hydrolyzing the compound of formula I under acidic conditions to obtain the compound of formula V, and
step (5): reductive amination of the compound of formula V with methylamine in the presence of a reductant, and salifying with hydrochloric acid to obtain the compound of formula VI,
wherein L is a leaving group, R is as defined in claim 1, M and n are as defined in claim 3, and X is halogen.
